(19) **Europäisches Patentamt European Patent Office Office européen des brevets**

(11) **EP 2 306 750 B1**

(12) **EUROPÄISCHE PATENTSCHRIFT**

(45) Veröffentlichungstag und Bekanntmachung des Hinweises auf die Patenterteilung:
**09.05.2012 Patentblatt 2012/19**

(51) Int Cl.:
*H04N 17/02* (2006.01)  *A61B 1/04* (2006.01)
*A61B 1/05* (2006.01)  *G01M 11/00* (2006.01)
*G02B 23/24* (2006.01)

(21) Anmeldenummer: **10010142.7**

(22) Anmeldetag: **22.09.2010**

(54) **Vorrichtung und Verfahren zum Prüfen von Endoskopen**

Apparatus and method for checking endoscopes

Procédé et dispositif destinés à la vérification d'endoscopes

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **01.10.2009 DE 102009043696**

(43) Veröffentlichungstag der Anmeldung:
**06.04.2011 Patentblatt 2011/14**

(73) Patentinhaber: **Karl Storz GmbH & Co. KG**
**78532 Tuttlingen (DE)**

(72) Erfinder:
• **Ehrhardt, André, Dr.**
**78573 Wurmlingen (DE)**

• **Glöggler, Bernhard**
**78532 Tuttlingen (DE)**
• **Beck, Gerd, Dr.**
**78573 Wurmlingen (DE)**
• **Tobien, Peter**
**78532 Tuttlingen (DE)**

(74) Vertreter: **Straub, Bernd**
**Karl Storz GmbH & Co. KG**
**Patentabteilung**
**Mittelstrasse 8**
**78532 Tuttlingen (DE)**

(56) Entgegenhaltungen:
**JP-A- 5 137 693  US-A- 5 142 359**
**US-B1- 6 361 490**

**Beschreibung**

Die vorliegende Erfindung betrifft eine Vorrichtung zur Prüfung von Endoskopen nach dem Oberbegriff von Anspruch 1 oder 2, sowie ein entsprechendes Verfahren zur Prüfung von Endoskopen.

Endoskope werden in vielfältiger Weise in Medizin und Technik eingesetzt, um das Innere von Hohlräumen oder andere schwer zugängliche Bereiche zu beobachten. Hierfür weisen Endoskope üblicherweise einen langerstreckten Schaft auf, der zur Einführung in einen Hohlraum geeignet ist, sowie einen Endoskopkopf, der einen visuellen Einblick gestattet oder einen Anschluss für eine Kamera oder eine Anzeigevorrichtung umfasst. Der Endoskopschaft kann, je nach vorgesehener Anwendung, starr, halbstarr oder flexibel ausgebildet sein.

Der Endoskopschaft enthält in der Regel einen Lichtleiter zur übertragung von Beleuchtungslicht, das in einer externen Lichtquelle erzeugt und in der Nähe des proximalen (d. h. beobachternahen) Endes des Endoskops eingekoppelt wird. Es ist aber auch möglich, dass das Beleuchtungslicht von einer innerhalb des Endoskops angeordneten Lichtquelle erzeugt wird. Eine solche Lichtquelle kann beispielsweise im Endoskopkopf, d. h., im proximalen Endbereich des Endoskops, angeordnet sein, aber auch im distalen (d. h. beobachterfernen) Bereich des Endoskops, d. h., im distalen Endbereich des Schafts; im letzteren Fall kann der Lichtleiter zur Übertragung des Beleuchtungslichts entfallen.

Ferner enthält das Endoskop ein Bildaufnahme- und Bildübertragungssystem. Hierzu ist im distalen Endbereich des Schafts ein Objektiv angeordnet, das ein Bild des beobachteten Objektfelds entwirft. Dieses Bild kann beispielsweise von einem ebenfalls im distalen Endbereich des Schafts angeordneten elektronischen Bildaufnehmer aufgenommen und über entsprechende Leitungen zur Weiterverarbeitung und Anzeige zum proximalen Ende des Endoskops übertragen werden. Das von dem Objektiv entworfene Bild kann aber auch über ein im Schaft aufgenommenes optisches Bildleitsystem zum proximalen Ende des Endoskops übertragen werden, wo es visuell betrachtet und/oder mit einer Videokamera aufgenommen werden kann. Ein solches optisches Bildleitsystem kann als Bündel von Lichtleitfasern oder auch als ein Relaislinsensystem, insbesondere eine Anordnung von Stablinsen, ausgebildet sein.

Für viele Anwendungen ist es ausreichend, breitbandiges Beleuchtungslicht zu erzeugen, beispielsweise über eine Xenon-Lichtquelle oder eine Weißlicht-LED, und ebenfalls in einem breiten optischen Spektralbereich zu beobachten, beispielsweise über einen visuellen Einblick oder mit Hilfe einer Farbvideokamera. In solchen Fällen werden optische Filter eingesetzt, um unerwünschte Strahlung von Komponenten des Endoskops und vom Objektfeld fernzuhalten, und um die spektrale Empfindlichkeitscharakteristik des bzw. der verwendeten Videochips zu korrigieren. Insbesondere werden zu diesem Zweck ein bevorzugt in einer externen Lichtquelle angeordnetes Wärmeschutzfilter eingesetzt sowie ein IR-Filter vor dem elektronischen Bildaufnehmer.

Insbesondere für bestimmte Anwendungen in der medizinischen Diagnostik ist es vorteilhaft, die Fluoreszenzeigenschaften von biologischem Gewebe auszunutzen, um Gewebeveränderungen zu erkennen. Die Beobachtung der von biologischem, insbesondere menschlichem Gewebe erzeugten Fluoreszenzstrahlung gestattet die Bestimmung der Verteilung und der Aktivität von Fluoreszenzstoffen im Gewebe. Da diese in sehr sensibler Weise vom Stoffwechsel und der Umgebung der Körperzellen abhängen, ermöglicht die Messung der Fluoreszenzstrahlung den Nachweis von Gewebeveränderungen, die durch Beobachtung des reflektierten Lichts nicht oder noch nicht erkennbar sind. Die Fluoreszenzstoffe können dabei körpereigene Stoffe (Autofluorophore) sein, die im Körpergewebe von Natur aus vorhanden sind. Es ist aber auch möglich, Fluoreszenzstoffe für die Diagnose zuzuführen, oder Stoffe zuzuführen, aus denen die Fluoreszenzstoffe durch Stoffwechselvorgänge entstehen. Da die Verteilung der Fluoreszenzstoffe jeweils von der Art, dem Zustand und der Umgebung der Zellen abhängig ist, ermöglicht die Fluoreszenz einen Rückschluss auf den Zustand des Gewebes und die Erkennung entarteten Gewebes.

Zur Diagnose von Veränderungen menschlichen Gewebes mit Hilfe der körpereigenen Fluoreszenz (Autofluoreszenz) ist eine Anregungsstrahlung im blauen Bereich des visuellen Spektrums mit Wellenlängen unterhalb von ca. 450 nm vorteilhaft. Die vom Gewebe abgegebene Fluoreszenzstrahlung weist ein relativ breites Spektrum auf und ist insbesondere im grünen Bereich des Spektrums beobachtbar.

Andererseits können zu Diagnose- oder Therapiezwecken auch Fluoreszenzstoffe zugeführt werden. Ein hierfür geeigneter Photosensibilisator ist beispielsweise 5-Aminolävulinsäure (5-ALA); ein auf 5-ALA basierendes Medikament wird von der Fa. GE Healthcare unter dem Namen HEXVIX® angeboten. Durch Stoffwechselvorgänge, die in praktisch allen Körperzellen stattfinden können, entsteht aus 5-ALA das fluoreszenzfähige Prutoporphyrin IX (PpIX), welches mit einer Anregungsstrahlung im Bereich von 405 nm zur Fluoreszenz im Bereich von ca. 635 nm, d. h., im roten Bereich des optischen Spektrums, angeregt werden kann.

Vorrichtungen und Verfahren zur Fluoreszenz-Diagnose sind bekannt. So wird beispielsweise in WO97/11636 eine Vorrichtung zur Diagnose mittels einer durch einen Photosensibilisator oder durch Eigenfluoreszenz hervorgerufenen Reaktion in biologischem Gewebe beschrieben, bei der ein Beleuchtungssystem zur Erzeugung von Fluoreszenzanregungslicht vorgesehen ist sowie eine lichtzuführende Einheit zur Beleuchtung eines zu beobachtenden Gewebebereichs mit dem Fluoreszenzanregungslicht. Das von dem Gewebebereich kommende Licht kann über eine Endoskopoptik aufgenommen und in einer proximalen Bildebene abgebildet werden, in der beispielsweise der Bildaufnehmer einer Videokamera angeordnet sein kann. Dabei wird ein Beleuchtungsfilter eingesetzt, das das breitbandige Beleuchtungslicht im Wellenlängenbereich unterhalb von ca. 440 nm möglichst ungeschwächt durchlässt, jedoch Licht mit Wellenlängen oberhalb von ca. 460 nm weitgehend abblockt. Andererseits ist im Beobachtungsstrahlengang ein Beobachtungsfilter eingesetzt, das das Beleuchtungslicht abblockt, um eine Überstrahlung der wesentlich schwächeren Fluoreszenzstrahlung zu vermeiden. Als Beobachtungsfilter wird gemäß WO97/11636 ein Langpassfilter mit einer Kante bei ca. 445 nm eingesetzt. Zur besseren Orientierung und für eine kontrastreichere Darstellung kann zusätzlich zur Fluoreszenzstrahlung auch ein geringer Anteil des reflektierten bzw. gestreuten Beleuchtungslichts vom Detektor aufgenommen und auf dem Videoschirm dargestellt werden. Hierfür sind die spektralen Eigenschaften des Anregungs- und des Beobachtungsfilters so aufeinander abgestimmt, dass in einem Überlappungsbereich ein für die gewünschte Darstellung geeigneter Anteil des Beleuchtungslichts vom Beobachtungsfilter durchgelassen wird.

Die in endoskopischen Systemen zur Fluoreszenzdiagnose verwendeten Anregungs- und Beobachtungsfilter können an unterschiedlichen Einbauorten angeordnet sein. Insbesondere bei solchen Endoskopen, bei denen im distalen Endbereich des Endoskopschafts ein elektronischer Bildaufnehmer angeordnet ist, muss der Beobachtungsfilter ebenfalls im distalen Endbereich des Schafts angeordnet sein. Da der Durchmesser des Schafts durch den Anwendungszweck in der Regel eng begrenzt ist, steht nur ein sehr enger Raum für die optischen und elektronischen Elemente zur Verfügung, Auch die Filter können daher oft nur Kantenlängen bzw. Durchmesser von wenigen Millimetern aufweisen, wobei praktisch die gesamte Fläche des Filters für die Transmission des Lichts benötigt wird. Es ist deshalb häufig nicht praktikabel, auf den Filtern deutlich erkennbare Kennzeichnungen anzubringen.

Da sich die Wellenlängenbereiche der Anregungsstrahlung bei Autofluoreszenz und bei durch einen Photosensibilisator induzierter Fluoreszenz, beispielsweise bei Verwendung von 5-ALA, geringfügig unterscheiden, sind hierfür geringfügig unterschiedliche Filter notwendig. Das gleiche gilt für die jeweils benötigten Beobachtungsfilter, die sich ebenfalls geringfügig unterscheiden, so dass diese nicht anhand ihrer visuell erkennbaren Farbe voneinander unterschieden werden können. Häufig werden die Filter aus Platzgründen und aus optischen Gründen, beispielsweise wenn die optimale Filterwirkung die Anordnung im konvergenten Strahlengang erfordert, direkt auf das Eintrittsfenster eines Bildaufnehmerchips aufgeklebt. Auch in diesem Fall ist nicht mehr erkennbar, welcher Art der Filter ist.

Ist ein solches Endoskop zur Fluoreszenzdiagnose bzw. -therapie zusammengebaut, so ist von außen nicht mehr erkennbar, welches Filter eingesetzt worden ist. Aufgrund der hygienischen Anforderungen muss das Endoskop auch insbesondere im Schaftbereich möglichst dicht ausgeführt sein, so dass die im Endoskop angeordneten Filter im Rahmen der üblichen Pflege und Wartung nicht mehr zugänglich sind. Dennoch ist es aus Gründen der Qualitätssicherung notwendig, feststellen zu können, welche Filter tatsächlich in einem Endoskop eingebaut sind. Verwechslungen können dazu fuhren, dass das Endoskopiesystem nicht bestimmungsgemäß verwendet werden kann, so dass die diagnostische Aussagekraft in Frage gestellt wäre.

Es ist bekannt, zur Kontrolle von Filtern in Endoskopen die Farbkanäle einer CCD-Kamera bei Beobachtung einer weißen Beobachtungsfläche auszuwerten. Die Genauigkeit dieses Verfahrens ist jedoch nicht ausreichend zur Unterscheidung von Filtern, die sich in ihren spektralen Eigenschaften nur geringfügig unterscheiden, wie etwa bei einem Lang- oder Kurzpassfilter mit geringfügig veränderter Kantenlage.

Aus der Patentschrift US 5,142,359 ist es bekannt, zu Einstellungszwecken bei elektronischen Endoskopen Farbtafeln einzusetzen. Eine solche Farbtafel weist Felder verschiedener Farben auf. Durch Beobachtung verschiedener Felder mit einem Endoskop ist es möglich, die spektrale Empfindlichkeit des Endoskops einschließlich des elektronischen Bildaufnehmers in den verschiedenen Farbkanälen einzustellen. Eine Verwendung von Farbtafeln zur Unterscheidung der in einem Fluoreszenzendoskop eingesetzten Filter wird in der US 5,142,359 nicht beschrieben. Hierfür müssten die Farben exakt auf die zu unterscheidenden Filter abgestimmt sein. Es sind jedoch nicht für alle in Frage kommenden Filter geeignete Farbstoffe verfügbar. Ein zusätzliches Problem bei der Prüfung von Fluoreszenzendoskopen stellt die Fluoreszenz des Trägermaterials, beispielsweise Papier, dar, auf das die Farbfelder aufgebracht werden. Die Unterscheidung von Filtern, deren Charakteristik sich nur geringfügig unterscheidet, insbesondere von in Fluoreszenzendoskopen eingesetzten Filtern, ist mit einer solchen Farbtafel daher nicht möglich.

Weiterhin ist es bekannt, die spektrale Transmission von Endoskopen mit Ililfe eines Spektrometers zu überprüfen. Hierbei wird Strahlung einer bekannten Wellenlängenverteilung in das Endoskop eingekoppelt und nach Durchgang durch die zu prüfenden optischen Elemente mit einem Spektrometer aufgenommen. Aus dem Verhältnis der aufgenommenen zu der eingekoppelten Intensität lässt sich die spektrale Transmission ermitteln, so dass auch die Transmission im Endoskop enthaltener Filter überprüft werden kann. Die Prüfung von Videoendoskopcn ist auf diese Weise nicht möglich. Zudem stellt ein Spektrometer einen erheblichen Kostenfaktor dar, und die Bedienung erfordert eine spezielle Schulung. Für den mutinemäßigen Einsatz im Rahmen einer Produktionskontrolle, insbesondere einer 100%-Prüfung ist dieses Verfahren daher nicht geeignet.

Aus der Offenlegungsschrift WO 98/11945 ist eine Vorrichtung zur Prüfung, Einstellung und/oder Schulung eines PDD- bzw. PDT-Systems bekannt, bei der ein Target vorgesehen ist, das zumindest eine Lichtquelle umfasst. Zur Anpassung des von der Lichtquelle emittierten Spektrums an das Emissionsspektrum eines Fluoreszenzmarkers ist ein Filter vorgesehen, das vor der Lichtquelle angeordnet ist. Hierfür muss der Durchlassbereich des Filters dem zu beobachtenden Fluoreszenzspektrum entsprechen. Für die Prüfung des Vorhandenseins bzw. der genauen spektralen Eigenschaften eines im Endoskop vorgesehenen Beobachtungsfilters ist die in der WO 98/11945 beschriebene Vorrichtung nicht geeignet. Es ist die Aufgabe der vorliegenden Erfindung, eine Vorrichtung und ein Verfahren zur Prüfung von Endoskopen anzugeben, die bzw. das auf einfache Weise eine Prüfung bzw. sichere Unterscheidung von im Endoskop eingebauten Filtern ermöglicht.

Diese Aufgabe wird durch eine Vorrichtung gemäß Anspruch 1 oder 2 sowie durch ein Verfahren gemäß Anspruch 14 oder 15 gelöst.

Vorteilhafte Weiterbildungen der Erfindung ergeben sich aus den Unteransprüchen.

Eine erfindungsgemäße Vorrichtung zur Prüfung eines Endoskops umfasst ein Target und eine Halterung zur Aufnahme des zu prüfenden Endoskops.

Das Target kann insbesondere als Scheibe mit einer im Wesentlichen ebenen Oberfläche oder auch als Teil einer Hohlkugel ausgebildet sein. Das Target umfasst mindestens ein Testfeld, das einen Teil einer Oberfläche oder auch die gesamte Oberfläche einer Seite des Targets einnehmen kann. Das Target ist insbesondere als mechanischer Träger für ein oder mehrere Testfelder ausgebildet und kann ein- oder mehrstückig sein.

Die Halterung ist zur Aufnahme des zu prüfenden Endoskops ausgebildet. Insbesondere kann die Halterung derart ausgestaltet sein, dass der distale Endbereich des Schafts eines zu prüfenden Endoskops in die Halterung eingesetzt werden kann. Hierdurch wird der distale Endbereich des Endoskopschafts ausreichend festgelegt, um während der Prüfung eine vorgegebene Position relativ zum Target einzunehmen. Bei einem flexiblen oder semiflexiblen Endoskop ist eine Festlegung des übrigen Teils des Endoskopschafts oder des Endoskopkopfs in der Regel nicht erforderlich. Bei einem starren Endoskop wird durch die Festlegung der Endoskopspitze das gesamte Endoskop fixiert. Zur Vermeidung einer unerwünschten mechanischen Beanspruchung des Endoskopschafts kann die Halterung auch eine Aufnahme für den gesamten Schaft oder für das gesamte Endoskop umfassen.

Die Halterung ist dabei derart ausgebildet, dass mit dem zu prüfenden Endoskop, wenn es in der Halterung aufgenommen ist, eine Beobachtung des Targets, insbesondere eine Beobachtung des mindestens einen Testfelds möglich ist. Die hierfür vorgegebene Postition relativ zum Target hängt von der Blickrichtung und vom Blickwinkel des Endoskops ab. Dabei muss die Position nicht genau vorgegeben sein. So kann je nach Blickwinkel eine Drehung um die Längsachse des Endoskops zulässig sein, solange noch das Testfeld im Blickfeld des Endoskops sichtbar ist; bei einem Geradeausblick-Endoskop kann auch eine freie Rotation um die Längsachse zulässig sein. Ebenso kann eine Verschiebung des Endoskopschafts in Längsrichtung möglich sein, ohne die Qualität der Beobachtung des Testfelds zu beeinträchtigen. Auch eine leichte Verkippung des distalen Endbereichs kann zulässig sein.

Das Testfeld enthält eine Testfilteranordnung, die aus einem oder mehreren hintereinander angeordneten optischen Filtern besteht. Diese können insbesondere in eine entsprechende Ausnehmung des Targets eingesetzt sein oder auch auf der Oberfläche des Targets gehalten, beispielsweise aufgeklebt sein.

Während die Beobachtung des Targets und damit des Testfelds mit dem Endoskop aus einer ersten Richtung erfolgt, ist die Testfilteranordnung aus einer zweiten Richtung, insbesondere aus einer der ersten entgegengesetzten Richtung, beleuchtbar. Hierdurch ist das durch die Testfilteranordnung transmittierte Licht mit dem Endoskop aufnehmbar. Die mit dem Endoskop beobachtete Helligkeit des Testfelds entspricht daher zumindest zum Teil dem von der Testfilteranordung durchgelassenen Licht. Auf diese Weise ist eine Helligkeit des Testfelds im transmittierten Licht feststellbar. Sofern das Testfeld bei der Beobachtung mit dem Endoskop nicht flächig aufgelöst wird, kann die Helligkeit die Gesamthelligkeit des durch das jeweilige Testfeld transmittierten Lichts sein. Bevorzugt ist jedoch, dass das Testfeld derart ausgebildet ist, dass es mit dem Endoskop flächenhaft aufgelöst beobachtet werden kann; in diesem Fall ist die Helligkeit die Flächenhelligkeit.

Zur Beleuchtung kann eine Lichtquelle eingesetzt werden, die von der Vorrichtung umfasst ist oder auch eine externe Lichtquelle. Die Lichtquelle kann auch dem Target zugehören.

So kann beispielsweise innerhalb des Testfelds eine Lichtquelle, beispielsweise eine lichtemittierende Diode (LED) angeordnet sein, vor der in Richtung auf das Endoskop die Testfilteranordnung angebracht ist. Ebenso kann innerhalb der Vorrichtung oder innerhalb des Targets eine Lichtquelle vorhanden sein, deren Strahlung über einen Lichtleiter, beispielsweise ein Bündel von Lichtleitfasern, zum Testfeld übertragen wird. Das Licht einer externen Lichtquelle kann direkt die Testfilteranordnung aus der zweiten Richtung beleuchten oder es kann über optische Übertragungsmittel, beispielsweise Spiegel oder Lichtleiter, zum Testfeld übertragen werden.

Die erfindungsgemäße Vorrichtung ist insbesondere zur Prüfung eines Endoskops vorgesehen, das eine Beobachtungsfilteranordnung aufweist, die das von dem Endoskop an der Spitze aufgenommene Licht in geeigneter Weise filtert, um eine für den Anwendungszweck geeignete Darstellung des endoskopischen Bildes zu erzielen. Insbesondere ist bei einem Fluoreszen-zendoskop eine Beobachtungsfilteranordnung notwen-dig, um zu vermeiden, dass durch die sehr helle Anregungsstrahlung die schwache Fluoreszenzstrahlung des Gewebes überstrahlt wird. Dabei kann die spektrale Charakteristik der Beobachtungsfilteranordnung vom jeweils beobachteten Fluoreszenzmodus abhängen. Solche Beobachtungsfilteranordnungen können aber auch beispielsweise das bei CCD-Bildaufnehmem übliche IR-Filter sein, das Licht im infraroten Spektralbereich ausfiltert, um mit einem Bildaufnchmer, der im Gegensatz zum menschlichen Auge auch im nahen Infrarot empfindlich ist, ein dem visuellen Eindruck entsprechendes Bild zu erhalten.

Die jeweils in dem betreffenden Endoskop vorgesehene Beobachtungsfilteranordnung weist eine vorgegebene spektrale Charakteristik auf, die in Abhängigkeit von der jeweiligen Anwendung, insbesondere dem jeweiligen Beobachtungsmodus, gewählt worden ist. Eine solche Soll-Charakteristik ist insbesondere die für ein eingesetztes Filter oder eine Filteranordnung spezifizierte Transmission in Abhängigkeit von der Wellenlänge, ggf. einschließlich der Charakteristik weiterer optischer Elemente, etwa eines Bildleiters. Die spektrale Soll-Charakteristik der Beobachtungsfilteranordnung kann insbesondere davon abhängen, ob Autofluoreszenz oder ALA-induzierte Fluoreszenz oder ob im reflektierten Licht beobachtet werden soll.

Um ein Endoskop, das eine Beobachtungsfilteranordnung aufweist, der eine spektrale Soll-Charakteristik zugeordnet ist, zu prüfen und insbesondere Abweichungen von der Soll-Charakteristik bzw. das Vorhandensein eines vorgesehenen Filters festzustellen, ist erfindungsgemäß die spektrale Charakteristik der Testfilteranordnung zu der Soll-Charakteristik der Beobachtungsfilteranordnung umgekehrt Dadurch führen auch geringe Abweichungen der spektralen Charakteristik der Beobachtungsfilteranordnung im Endoskop von der Soll-Charakteristik zu einer deutlichen Veränderung der mit dem Endoskop beobachteten Helligkeit des Testfelds. Ebenso ist das Fehlen eines Filters der Beobachtungsfilteranordnung oder das Vorhandensein eines Filters mit geringfügig unterschiedlicher spektraler Transmission durch einen maximalen Kontrast erkennbar.

Eine gegenüber einem anderen Filter umgekehrte spektrale Charakteristik eines Filters ist insbesondere eine solche, bei der das eine Filter in spektralen Bereichen durchlässig ist, in denen das andere Filter blockiert, und in spektralen Bereichen blockiert, in denen das andere Filter durchlässig ist. Bei einem Kantenfilter bedeutet dies, dass beide Filter nahezu die gleiche Kantenlage besitzen, jedoch eines ein Langpass- und das andere ein Kurzpassfilter ist. Es kann dann beispielsweise in einem gewissen Spektralbereich bei jeder Wellenlänge $\lambda$ für die Transmission $T_1(\lambda)$ des einen Filters gelten:

$$T_1(\lambda) \approx 1 - T_2(\lambda + \Delta\lambda),$$

wobei $T_2(\lambda)$ die Transmission des anderen Filter und $\Delta\lambda$

der Abstand zwischen den 50%-Punkten der beiden Filter ist, der sich aus der technisch erreichbaren Kantensteilheit und der Zweckbestimmung der Filter ergibt. Allerdings muss die Wellenlänge der Kante des Kurzpassfilters nicht genau die gleiche sein wie die des Langpassfilters, vielmehr können in einem gewissen Bereich beide Filter durchlässig sein oder beide blockieren. Welcher Bereich dabei zulässig ist, hängt von der gewünschten Prüfgenauigkeit bzw. den erwarteten oder zu unterscheidenden Abweichungen der spektralen Transmission von der Solltransmission ab. Ebenso ist bei Filtern mit einer Transmissionsfunktion, die kontinuierlich Werte zwischen 0 und 100% annimmt, die o. g. Beziehung nur näherungsweise gültig, wobei der Bereich zulässiger Abweichungen von der gewünschten Prüfgenauigkeit bzw. Trennschärfe zwischen unterschiedlichen Filtern abhängt, sowie von der Steilheit der Transmissionskurve in den Bereichen, in denen diese gegenläufig ist. Das Gleiche gilt auch für Anordnungen aus mehreren hintereinander geschalteten Filtern. Dabei bleiben generell Wellenlängenbereiche, die außerhalb des Empfindlichkeitsbereichs des jeweiligen Detektors liegen, außer Betracht, also bei visueller Betrachtung Wellenlängenbereiche außerhalb des visuellen Bereichs und bei Verwendung eines elektronischen Bildaufnehmers solche außerhalb des Empfindlichkeitsbereich des Bildaufnehmers.

Insbesondere kann beispielsweise die Beobachtungsfilteranordnung für ALA-induzierte Fluoreszenz einen Transmissionsgrad $T_B(\lambda)$ aufweisen mit

$$T_B(\lambda) \leq 0{,}1\ \% \text{ für } 380\ nm < \lambda < 430\ nm,$$

$$T_B(\lambda) \cong 50\ \% \text{ für } \lambda = 444\ nm,$$

$$T_B(\lambda) \geq 96\ \% \text{ für } 450\ nm < \lambda < 800\ nm.$$

Der Transmissionsgrad $T_A(\lambda)$ der Testfilteranordnung kann dann beispielsweise für ein Endoskop zur Beobachtung ALA-induzierter Fluoreszenz bzw. für den ALA-Beobachtungsmodus eines Fluoreszenzendoskops mit mehreren Beobachtungsmoden näherungsweise gegeben sein durch:

$$T_A(\lambda) \geq 95\ \% \text{ für } \lambda < 410\ nm,$$

$$T_A(\lambda) \cong 50\ \% \text{ für } \lambda = 430\ nm,$$

$$T_A(\lambda) \leq 1\ \% \text{ für } \lambda > 450\ nm.$$

Die Beobachtungsfilteranordnung für Autofluoreszenz kann beispielsweise einen Transmissionsgrad $T_B(\lambda)$ aufweisen mit

$$T_B(\lambda) \leq 0{,}1\ \% \text{ für } 380\ nm < \lambda < 455\ nm,$$

$$T_B(\lambda) \cong 50\ \% \text{ für } \lambda = 471\ nm,$$

$T_B(\lambda) \geq 96\,\%$ für $475$ nm $< \lambda < 800$ nm.

Der Transmissionsgrad $T_A(\lambda)$ der Testfilteranordnung kann dann beispielsweise für ein Endoskop zur Beobachtung von Autofluoreszenz (AF) bzw. für den AF-Beobachtungsmodus eines Fluoreszenzendoskops mit mehreren Beobachtungsmoden näherungsweise gegeben sein durch:

$T_A(\lambda) \geq 95\,\%$ für $\lambda < 430$ nm,

$T_A(\lambda) \cong 50\,\%$ für $\lambda = 450$ nm,

$T_A(\lambda) \leq 1\,\%$ für $\lambda > 470$ nm.

Eine erfindungsgemäße Vorrichtung zur Prüfung eines Endoskops für Fluoreszenzbeobachtungen, insbesondere für die Fluoreszenzdiagnose, umfasst ebenfalls ein Target mit mindestens einem Testfeld sowie eine Halterung zur Aufnahme des Endoskops zur Beobachtung des mindestens einen Testfelds aus einer ersten Richtung, wobei das Testfelds eine Testfilteranordnung enthält, die aus einer zweiten Richtung heleuchtbar ist, so dass dem Testfeld eine mit dem Endoskop beobachtbare Helligkeit im durch die Testfilteranordnung transmittierten Licht zugeordnet werden kann. Das Endoskop weist für Fluoreszcnzbeobachtungen eine Beobachtungsfilteranordnung mit einer vorgegebenen Soll-Charakteristik auf. Ferner ist dem Fluoreszenzendoskop eine Beleuchtungsfilteranordnung zugeordnet, die im Endoskop angeordnet sein kann, bevorzugt jedoch zumindest teilweise in einer für Fluoreszenzbeobachtungen mit dem Fluoreszenzendoskop zu verwendenden externen Lichtquelle angeordnet ist. Eine solche Beleuchtungsfilteranordnung ist insbesondere bei Fluoreszenzendoskopen notwendig, um die Fluoreszenzanregungsstrahlung in das Gewebe einzukoppeln, dabei jedoch in demjenigen Wellenlängenbereich, in dem die Fluoreszenzstrahlung emittiert wird, keine oder nur sehr wenig Strahlung durchzulassen, die die beobachtete Fluoreszenzstrahlung überdecken würde; zudem soll möglichst wenig Wärme in das Endoskop und in das Gewebe eingebracht werden. Erfindungsgemäß entspricht die spektrale Charakteristik der Testfilteranordnung der spektralen Charakteristik der Belcuchtungsfilteranordnung Hierdurch ist eine ausreichend sichere Prüfung der Beobachtungsfilteranordnung möglich.

Dass die Testfilteranordnung im Wesentlichen die gleiche spektrale Charakteristik wie die dem zu prüfenden Endoskop zugeordnete Beleuchtungsniteranordnung hat, hat den besonderen Vorteil, dass die gleichen Filter, die für die Beleuchtungsfilteranordnung verwendet werden, auch in der erfindungsgemäßen Vorrichtung eingesetzt werden können. Hierdurch ist eine einfache und kostengünstige Beschaffung möglich.

Gemäß einer weiteren bevorzugten Ausführungsform der Erfindung besteht die Testfilteranordnung des mindestens einen Testfelds aus einer Mehrzahl von Filtern, die insbesondere im Lichtweg hintereinander angeordnet sind. Hierdurch ist es möglich, nahezu jede gewünschte spektrale Transmissionsfunktion zu realisieren. Insbesondere können Kanten-, Block-, Grau-, Bandpass- und/oder andere Filter je nach Art des zu prüfenden Endoskops miteinander kombiniert werden. Ebenso können verschiedene im zu prüfenden Endoskop bzw. in verschiedenen zu Varianten eines zu prüfenden Endoskops vorgesehene Filter in einer Filteranordnung miteinander kombiniert werden.

In einer weiteren bevorzugten Ausführungsform umfasst das Target mindestens ein Referenzfcld. Das Referenzfeld enthält keine Filteranordnung oder aber eine solche, bei der die mit dem Endoskop beobachtete Helligkeit nicht wesentlich von der spektralen Charakteristik der Beobachtungsfilteranordnung abhängt. Das Referenzfeld kann auch beispielsweise Graufilter zur Anpassung der Helligkeit enthalten. Ebenso kann das Referenzfeld eine Kombination aus Anregungs- und/oder Beobachtungsfiltern des zu prüfenden Endoskops enthalten. Durch einen Vergleich der Helligkeit des mindestens einen Testfelds mit der des Referenzfelds ist eine einfache Prüfung der spektralen Charakteristik der Beobachtungsfilteranordnung bzw. eine einfache Feststellung des Vorhandenseins eines Beobachtungsfilters möglich. Zudem kann mit Hilfe des Referenzfelds eine Einstellung der Lichtquelle und/oder des Bildaufnehmers erfolgen, und Fehler beispielsweise aufgrund fehlerhafter Einstellung von Beleuchtungslichtstärke, Belichtungzeit, Verstärkungsfaktor usw. können ausgeschlossen werden.

Insbesondere ist es vorteilhaft, wenn das Target eine Mehrzahl von Testfeldern aufweist, die jeweils eine Testfilteranordnung enthalten. Die jeweiligen Testfilteranordnungen können gleich oder unterschiedlich voneinander sein. Ebenso kann eine Mehrzahl von Referenzfeldem vorhanden sein. Eine Mehrzahl von Feldern erlaubt eine sicherere Feststellung der zu prüfenden Eigenschaften des Endoskops. Insbesondere können Fehler aufgrund beispielsweise einzelner dunkler Fasern eines Bildleiters oder aufgrund eines Helligkeitsabfalls am Rand des Bildfelds sicherer ausgeschlossen werden. Ebenso kann durch Vergleich der Helligkeit mehrerer Testfelder, die unterschiedliche Testfilteranordnungen aufweisen können, untereinander ein sichereres Testergebnis erzielt werden. Die Testfelder können auch in Form eines Prüfmusters angeordnet sein, wobei das Prüfmuster gleichzeitig die Prüfung weiterer optischer Eigenschaften, wie beispielsweise der Verzeichnung oder einer Randabschattung, ermöglichen kann.

Gemäß einer bevorzugten Ausführungsform der Erfindung sind die spektralen Charakteristiken der Testfilteranordnungen mindestens zweier Testfelder voneinander unterschiedlich. Dies hat den besonderen Vorteil, dass verschiedene Abweichungen der spektralen Charakteristik der Beobachtungsniteranordnung von der Soll-Charakteristik sicher festgestellt werden können. So können beispielsweise zur Überprüfung der Kantenlage eines Kantenfilters in verschiedenen Testfeldern Testfiltermordnungen mit unterschiedlichen Kantenlagen eingesetzt werden, um sowohl für eine Verschiebung der Kantenlage zu längeren Wellenlängen als auch für eine Verschiebung zu kürzeren Wellenlängen die jeweils maximale Empfindlichkeit bzw. den maximalen Kontrast zum Referenzfeld zu erzielen.

Bei Fluoreszenzbeobachtungen, insbesondere bei der Fluoreszenzdiagnose zur Erkennung von Veränderungen menschlichen Gewebes, kann je nach Anwendungszweck eine Beobachtung der Autofluoreszenz, der ALA-induzierten Fluoreszenz, der durch andere Photosensibilisatoren induzierten Fluorescenz oder auch eine Beobachtung im reflektierten bzw. gestreuten Licht, beispielsweise im Weißlicht, in einem Anteil des blauen Anregungslichts oder in einem hierfür beigemischten zusätzlichen Beleuchtungslicht, vorteilhaft sein. Besonders vorteilhaft kann eine Kombination mehrerer Beobachtungsverfuhren sein, die gleichzeitig oder in zeitlicher Folge durchgeführt werden, um eine besonders kontrastreiche Darstellung des Gewebes zu erhalten. Deshalb sind bei Fluoreszenzendoskopen für die unterschiedlichen Beobachtungsverfahren häufig mehrere Varianten im Gebrauch bzw. mehrere Betriebsweisen möglich. Um das Vorhandensein bzw. Nichtvorhandensein der hierbei eingesetzten Filter, die sich oft nur geringfügig unterscheiden, sicher erkennen zu können bzw. ein eingesetztes Filter sicher identifizieren zu können, ist gemäß einer weiteren bevorzugten Ausführungsform der Erfindung die spektrale Charakteristik der Testfilteranordnung mindestens eines Testfelds im Wesentlichen gleich der spektralen Charakteristik der einer dem zu prüfenden Endoskop in einer ersten Variante bzw. einem ersten Betriebszustand zugeordneten Beleuchtungsfilteranoronung und die spektrale Charakteristik der Testfilteranordnung mindestens eines weiteren Testfelds im Wesentlichen gleich der einer dem zu prüfenden Endoskop in einer anderen Variante bzw. einem anderen Betriebszustand zugeordneten Beleuchtungsfilteranordnung; auch Kombinationen der verschiedenen verwendeten Anregungsfilter sind möglich. Hierdurch kann auf einfache Weise eine Testvorrichtung realisiert werden, die eine sichere Unterscheidung der Varianten bzw. Betriebszustände des Endoskops ermöglicht.

Weiterhin ist es vorteilhaft, wenn die Test- und/uder Referenzfelder unterschiedliche geometrische Formen aufweisen. So kann beispielsweise die Form der Testfelder untrreiniander gleich, aber unterschiedlich von den Referenzfeldern sein. Ebenso kann die Form verschiedener Testfelder unterschiedlich sein, insbesondere solcher mit unterschiedlichen spektralen Charakteristiken der betreffenden Testfilteranordnungen. Die geometrische Form kann dabei beispielsweise die einer Kreisscheibe, einer Ellipse, eines Quadrats bzw. einer Raute, eines Sechsecks oder eine andere Form sein.

Dies hat den Vorteil, dass es leicht erkennbar ist, welcher Testfiltermordnung eine beobachtete Helligkeit eines Testfelds und damit ein entsprechendes Prüfungsergebris zuzuordnen ist. Die eindeutige Zuordnung der Testfelder wird daher nicht durch eine unbemerkte Rotation des Endoskops um die Längsachse gestört. Dies ist insbesondere bei elektronischen Endoskopen wichtig, bei denen je nach Rotation um die Längsachse ein Gegenstand im Objektfeld einmal aufrecht und einmal umgekehrt erscheinen kann. Bei einer automatisierten Durchführung einer Endoskopprüfung mit der erfindungsgemäßen Vorrichtung wird durch eine unterschiedliche Formgebung der Test- und/oder Referenzfelder zudem ein sichereres Ergebnis erzielt.

Die Test- und/oder Referenzfelder können gemäß einer weiteren Ausführungsform der Erfindung auch in Form eines Musters angeordnet, bei dem einer bestimmten Position innerhalb des Musters eine bestimmte Testfiltercharakteristik zugeordnet ist. So kann beispielsweise ein Testfeld von einem Ring von Referenzfeldern umgeben sein, oder mehrere Testfelder in mehreren Ringen angeordnet sein. Auch hierdurch kann eine einfache und sichere Zuordnung der jeweiligen Felder ermöglicht werden.

In einer weiteren bevorzugten Ausführungsform der Erfindung kann eine Mehrzahl von Test- und/oder Referenzfeldern von einer gemeinsamen Lichtquelle beleuchtet werden. Dies hat den Vorteil, dass weitgehend unabhängig von der tatsächlichen Lichtleistung der Lichtquelle der Kontrast zwischen Test- und/oder Referenzfeldern eine sichere Prüfung des Endoskops gestattet. Darüber hinaus ermöglicht ein solcher Aufbau eine kostengünstige Realisierung, da nur eine einzige Lichtquelle erforderlich ist.

Weiterhin ist es vorteilhaft, wenn das Target lichtundurchlässig ausgebildet ist und mindestens ein Test- und/oder Referenzfeld als lichtdurchlässiger Bereich des Targets ausgebildet ist. Hierdurch wird eine besonders kontrastreiche Beobachtung der Test- und/oder Referenzfelder ermöglicht. Ein besonders einfacher Aufbau der erfindungsgemäßen Vorrichtung ist dadurch möglich, dass die Testfelder und die Referenzfelder als Durchbrüche eines scheiben- oder schalenförmigen Targets ausgebildet, in die die jeweilige Testfilteranordnung eingesetzt ist. Die Beobachtung erfolgt dann von der einen Seite der Scheibe bzw. Schale, während von der anderen Seite eine oder mehrere Testfilteranordnungen und/oder die Referenzfelder beleuchtet werden können.

Als Lichtquelle kann insbesondere eine externe Lichtquelle eingesetzt werden, die für die Beobachtung mit dem Endoskop vorgesehen ist. Das Lichtleitkabel, das für die Beobachtung an das Endoskop angeschlossen wird, kann in bevorzugter Weise derart an die erfindungsgemäße Prüfvorrichtung angeschlossen werden, dass der von der Endfläche des Lichtlcitkabels ausgehende Lichtkegel mehrere oder alle Test- und Referenzfelder erleuchtet. Als Lichtquelle ist eine solche Lichtquelle geeignet, die in dem Wellenlängenbereich, der für die Unterscheidung bzw. Prüfung der im Endoskop einzusetzenden Filter genutzt wird, eine ausreichende Helligkeit erzeugt. Insbesondere ist hierfür eine Weißlichtquelle vorteilhaft, beispielsweise eine Xenon-Lichtquelle. In vorteilhafter Weise kann für die Prüfung eines Fluoreszenzendoskops eine Lichtquelle verwendet werde, die für die Fluoreszcnzendoskopie gemeinsam mit dem bzw. den zu prüfenden Typen von Endoskopen eingesetzt wird. Sofern in der Lichtquelle ein Beleuchtungsfilter vorhanden ist, kann es notwendig sein, dieses vor der Verwendung für die Endoskopprüfung zu entfernen.

Weiterhin kann ein Linsenmodul vorhanden sein, das zur Erzeugung eines quasi-parallelen Strahlengangs und damit zur gleichmäßigen Beleuchtung der Test- und/oder der Referenzfelder ausgebildet ist. Insbesondere kann das Linsenmodul eine asphärische Linse sein, die an den Öffnungswinkel des Beleuchtungslichtkegels sowie an die Dimensionen des Targets angepasst ist und deren Brennpunkt in der Lichtaustrittstläche eines in die Vorrichtung eingesetzten Beleuchtungslichtleiters angeordnet wird.

Gemäß einer weiteren bevorzugten Ausführungsform ist in mindestens einem Test- und/oder Referenzfeld eine Streuscheibe vorhanden. Dies hat den Vorteil einer gleichmäßigeren Helligkeit, so dass unabhängig von der genauen Position der Endoskopspitze eine genaue Helligkeitsbestimmung möglich ist. In besonders bevorzugter Weise sind in allen Testfeldern Streuscheiben eingesetzt, bevorzugt auf der endoskopseitigen Endfläche der Teitfilteranordnung. Ebenso sind bevorzugt in allen Referenzfeldern Streuscheiben eingesetzt. Dies hat den weiteren Vorteil, dass alle Test- und/oder Referenzfelder stets mit einer von der genauen Ausrichtung des Endoskops unabhängigen Helligkeit beobachtet werden können und damit eine sichere Helligkeitsbestimmung bzw. Kontrastbestimmung zwischen den Helligkeiten der einzelnen Test- und Referenzfeldern möglich ist.

Gemäß einer weiteren bevorzugten Ausführungsform weist die Vorrichtung ferner ein lichtdichtes Gehäuse auf, das einen Hohlraum umschließt, in dem das Target angeordnet ist und der durch die Halterung lichtdicht abgeschlossen ist. Dies hat den Vorteil, dass Streustrahlung oder Raumlicht vermieden werden, die die Helligkeits- bzw. Kontrasterkennung stören könnten. Insbesondere kann der Hohlraum dann lichtdicht abgeschlossen sein, wenn in die Halterung ein zu prüfendes Endoskop eingesetzt ist.

Weiterhin ist es vorteilhaft, wenn die Halterung als auswechselbarer Adapter ausgebildet ist, der in das lichtdichte Gehäuse einsetzbar ist. Dabei können unterschiedliche Adapter vorgesehen sein, die sich durch den Durchmesser einer Bohrung, in die die Endoskopspitze einzusetzen ist, durch den Winkel der Bohrung zur Richtung zum Target oder durch weitere Merkmale, etwa zur Festlegung einer rotatorischen Ausrichtung des Endoskops bei Seitblickendoskopen, unterscheiden. Hierdurch wird es ermöglicht, unterschiedliche Endoskope mit unterschiedlichen Durchmesser, Blickwinkeln, Blickrichtungen usw. in die erfindungsgemäße Vorrichtung einzusetzen und zu prüfen.

Ein erfindungsgemäßes Verfahren zur Prüfung eines Endoskops, das eine Beobachtungsfilteranordnung mit einer vorgegebenen Soll-Charakteristik aufweist, umfasst die folgenden Schritte:

- Bereitstellen eines Targets mit mindestens einem Testfeld, das eine Testfilteranordnung enthält, wobei die spektrale Charakteristik der Testfilteranordnung zu der Soll-Charakteristik der Beobachtungsfiltenunurdnung umgekehrt ist,

- Anordnen des Endoskops in einer Halterung zur Beobachtung des mindestens einen Testfelds aus einer ersten Richtung,

- Beleuchten der Testfilteranordnung aus einer zweiten Richtung,

- Bestimmen einer Helligkeit des Testfelds mit dem Endoskop und

- Vergleichen der Helligkeit des Testfelds mit einer Soll-Helligkeit.

Ein erfindungsgemäßes Verfahren zur Prüfung eines Endoskops für Fluoreszenzbeobachtungen, das eine Beobachtungsfilteranordnung mit einer vorgegebenen Soll-Charakteristik aufweist, wobei dem Endoskop eine Beleuchtungsfilteranordnung zugeordnet ist, umfasst die folgenden Schritte:

- Bereitstellen eines Targets mit mindestens einem Testfeld, das eine Testfilteranordnung enthält, wobei die spektrale Charakteristik der Testfilteranordnung der Charakteristik der Beleuchtungsfilteranordnung entspricht,

- Anordnen des Endoskops in einer Halterung zur Beobachtung des mindestens einen Tcstfelds aus einer ersten Richtung,

- Beleuchten der Testfilteranordnung aus einer zweiten Richtung,

- Bestimmen einer Helligkeit des Testfelds mit dem Endoskop, und

- Vergleichen der Helligkeit des Tcstfelds mit einer Soll-Helligkeit.

Ein erfindungsgemäßes Verfahren kann sowohl visuell als auch teilweise oder ganz automatisch ablaufen. Dabei kann es insbesondere vorteilhaft sein, die Bestimmung der Helligkeit des Testfelds und den Vergleich mit einer vorbestimmten Soll-Helligkeit über eine automatische Bildverarbeitung ablaufen zu lassen. Hierfür kann eine Steuer- und Auswertevorrichtung vorgesehen sein zur Steuerung der Helligkeit der Lichtquelle und zur Auswertung der Helligkeit des Testfelds. Hierdurch kann schnell und sicher ein eindeutiges Prüfungsergebnis erzielt werden.

Insbesondere ist es dabei vorteilhaft, wenn das Target neben dem Testfeld zumindest ein Referenzfeld aufweist, dessen Helligkeit von der spektralen Charakteristik der Beobachtungsfilteranordnung nicht oder nur wenig abhängt, so dass durch einen Vergleich der Helligkeit des Testfelds mit der des Referenzfelds auf besonders einfache und sichere Weise die Eigenschaften der Beobachtungsfilteranordnung geprüft werden können. Die Soll-Helligkeit, mit der die Helligkeit des Testfelds verglichen wird, ist dann die Helligkeit des Referenzfelds. Die Soll-Helligkeit kann aber auch unabhängig hiervon vorbestimmt sein; ebenso kann die Soll-Helligkeit, die zur Aufwertung der Prüfung herangezogen wird, die Differenz der Helligkeiten zweier Testfelder sein.

Zur Durchführung des Verfahrens ist es dabei besonders vorteilhaft, wenn das Testfeld und das Referenzfeld durch einfache geometrische Merkmale, wie etwa Form oder Größe, voneinander unterschieden werden können. Ebenso kann die Anordnung mehrerer Test- und/oder Referenzfelder dazu dienen, diese eindeutig zu erkennen. Es ist dann nicht mehr nötig, eine Orientierung des Endoskops in der Halterung vorzugeben, sondern eine Identifizierung der Test- bzw. Referenzfelder ist allein durch Auswertung des vom Endoskop gelieferten Bilds möglich.

In einer bevorzugten Ausführungsform dient das erfindungsgemäße Verfahren zur Unterscheidung der für die verschiedenen Beobachtungsverfahren der Fluoreszenzendoskopic eingesetzten Beobachtungsfilter. Hierfür sind mehrere Testfelder vorhanden, die jeweils Testfilteranordnungen aufweisen, die den Beleurbtungsfiltern entsprechen, die für die jeweiligen Beobachtungsverfahren eingesetzt werden. Durch Vergleich der Helligkeiten der auf dem Target nebeneinander sichtbaren Testfelder untereinander sowie mit ebenfalls auf dem Target sichtbaren Referenzfeldern ist eine schnelle, einfache und sichere Erkennung des im Endoskop eingesetzten Beobachtungsfilters möglich.

Die vorstehende beschriebene Vorrichtung, die insbesondere zur Prüfung von Endoskopen vorgesehen ist, kann auch für die Prüfung anderer optischer Apparate oder Endoskop-Komponenten, wie etwa Mikroskope, Periskope, Objektive, Video Module usw. geeignet sein. Ebenso kann das erfindungsgmäße Verfahren auch für die Prüfung solcher optischer Apparate eingesetzt werden. Die erfindungsgemäße Prüfvorrichtung und das erfindungsgemäße Prüfverfahren können auch für die Prüfung bzw. Identifikation von Filtern verwendet werden, ohne dass diese Filter in eine Optik eingebaut sein müssen. Dies ist insbesondere dann vorteilhaft wenn solche Filter keine deutliche Beschriftung tragen und auch sonst keine einfache Möglichkeit zur Identifikation besteht. Es versteht sich ferner, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:

Fig. 1a ein Ausführungsbeispiel einer erfindungsgemäßen Vorrichtung in Längsschnitt;

Fig. 1b das Ausfuhrungsbeispiel der Fig. 1a, aus Richtung des Endoskop-Anschlusses gesehen;

Fig. 2 ein als Target dienendes Filtermodul in teilweise aufgeschnittener Darstellung;

Fig. 3 eine schematische Darstellung einer erfindungsgemäßen Vorrichtung im Prüfbetrieb;

Fig. 4 beispielhafte Filtercharakteristiken für zwei Fluoreszenzmoden;

Fig. 5a die Filtercharakteristiken des Anregungs- und des Beobachtungsfilters für Autofluoreszenz;

Fig. 5b die Filtercharakteristiken des Anregungs- und des Beobachtungsfilters für ALA-induzierte Fluoreszenz;

Fig. 5c die Filtercharakteristiken des Anregungsfilters für ALA-induzierte Fluoreszenz und des Beobachtungsfilters für Autofluoreszenz;

Fig. 5d die Filtercharakteristiken des Anregungsfilters für AF-induzierte Fluoreszenz und des Beobachtungsfilters für ALA-induzierte Fluoreszenz;

Fig. 6a bis 6c beispielhaft das mit dem Endoskop beobachtbare Bild des Targets.

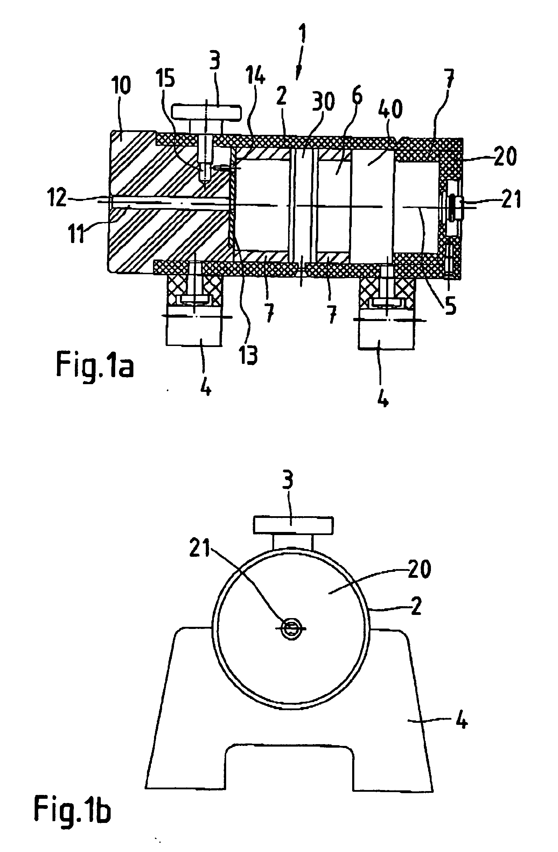

Gemäß Fig. 1 a und 1b umfasst eine erfindungsgemäße Prüfvorrichtung 1 im Ausführungsbeispiel ein rohrförmiges Gehäuse 2, in das an einem ersten Ende ein Adapter 10 einsetzbar ist. Der Adapter 10 weist eine Bohrung 11 auf, in die das distale Ende des Schafts eines zu prüfenden Endoskops einsetzbar ist. Hierfür besitzt die Bohrung 11 einen Durchmesser, der geringfügig größer ist als der Außendurchmesser des distalen Endbereichs des Endoskopschafts. Am äußeren Ende der Bohrung 11 kann eine Fase 12 oder eine trichterförmige Einsenkung angebracht sein, um die Spitze des Endoskops leichter einführen zu können. Am inneren Ende der Bohrung ist eine Platte 13 befestigt, die eine Bohrung 14 aufweist, die einen geringeren Durchmesser als die Bohrung 11 hat, so dass die Platte 13 einen Anschlag für das distale Ende des Endoskops bildet. Der Adapter 10 kann beispielsweise durch eine Schraube 3, insbesondere eine Rändelschraube, oder einen gefederten Stift, die bzw. der in eine Bohrung 15 des Adapters 10 eingreift, gesichert werden. An einen zweiten Ende ist das rohrförmige Gehäuse 2 durch eine Lichtanschlussplatte 20 abgeschlossen, die einen Lichtanschluss 21 für das Kabel einer externen Lichtquelle aufweist. Ferner weist die Prüfvorrichtung 1 Füße 4 auf zur Gewährleistung eines sicheren Stands. Die Bohrung 11 und der Lichtanschluss 21 können koaxial zu der Längsachse 5 des rohrförmigen Gehäuses 2 angeordnet sein. Diese Anordnung ist insbesondere für die Prüfung von Geradeausblick-Endoskopen geeignet. Für die Prüfung von Schrägblickendoskopen kann die Bohrung 11 auch schräg zur Längsachse 5 gerichtet sein, für die Prüfung von Scitblick- oder Rückblickendoskopen können beispielsweise Bohrungen im Gehäuse 2 vorgesehen sein, oder der Adapter kann selbst rohrförmig mit entsprechenden Bohrungen ausgestaltet und an das erste Ende des Gehäuses 2 ansetzbar sein.

Das Gehäuse 2 ist zusammen mit Adapter 10 und Lichtanschlussplatte 20 lichtdicht abgeschlossen. Wenn in die Bohrung 11 ein Endoskop mit einem der Bohrung entsprechenden Durchmesser eingesetzt ist und am Lichtanschluss 21 ein Lichtanschlusskabel angeschlossen ist, ist der dadurch umschlossene Hohlraum 6 gegen das Eindringen von Fremdlicht geschützt. Sofern weitere Bohrungen zum Einsetzen von Endoskopen anderer Blickwinkel, Blickrichtungen oder Schaftdurchmesser vorhanden sind, können diese lichtdicht verschließbar sein. Das gleiche gilt für ggf. vorhandene weitere Lichtarnschlüsse.

Innerhalb des Hohlraums 6 ist ein Filtermodul 30 angeordnet, das als Target dient und von einem in die Bohrung 11 bis zu dem durch die Platte 13 gebildeten Anschlag eingesetzten Endoskop beobachtbar ist. Ferner kann innerhalb des Hohlraums 6 ein Linsenmodul 40 angeordnet sein, das aus dem von einem am Lichtanschluss 21 angeschlossenen Lichtleitkabel austretenden divergenten Lichtbündel ein zur Längsachse 5 paralleles Lichtbündel macht. Hierdurch kann eine gleichmäßiger Ausleuchtung der Testfelder erzielt werden. Häufig kann aber auch auf ein Linsenmodul verzichtet werden. Durch Hülsen 7 werden die jeweiligen Elemente in ihrer Position innerhalb des Gehäuses gehalten. Um die Prüfung unterschiedlicher Endoskoptypen zu ermöglichen, kann das Filtermodul auswechselbar sein, wobei jeweils ein Filtermodul mit den für die Kontrolle eines bestimmten Filter- bzw. Endoskoptyps geeigneten Filtern bestückt sein kann.

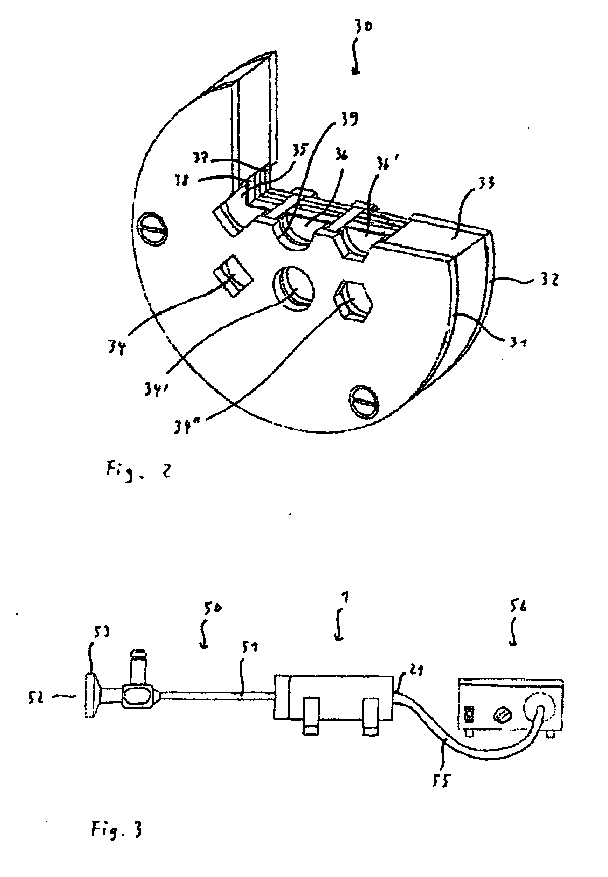

Wie in Fig. 3 gezeigt, umfasst das Filtermodul 30 eine erste Deckplatte 31, die in Richtung auf das Endoskop angeordnet ist, eine zweite Deckplatte 32, die in Richtung auf den Lichtanschluss angeordnet ist, und eine dazwischenliegende Scheibe 33. Die erste Deckplatte 31, die zweite Deckplatte 32 und die Scheibe 33 weisen miteinander korrespondierende Durchbrüche 39 auf, in denen optische Elemente angeordnet sind, und durch die ein Lichtweg ermöglicht wird. In der in Fig. 3 gezeigten Anordnung sind insgesamt 6 Durchbrüche vorgesehen, die in zwei Reihen zu je drei Durchbrüchen angeordnet sind. Dabei bildet die in Fig. 3 untere Reihe drei Hell-Referenzfelder 34, 34' 34", während die obere Reihe ein Dunkel-Referenzteld 35 und zwei Testfelder 36, 36' bildet. Die Test- und die Referenzfelder sind hier also in Form einer 2 x 3-Matrix angeordnet. Zumindest in der ersten Deckplatte 31, die als Blende dient, sind die Durchbrüche 39 in unterschiedlichen geometrischen Formen ausgebildet, beispielsweise als Raute, als Kreis und als Sechseck.

Innerhalb der Durchbrüche 39 sind optische Elemente angeordnet, die insbesondere optische Filter 37 und Streuscheiben 38 umfassen können. Die Streuscheiben 38 sind in Richtung auf das Endoskop angeordnet, um eine vom Blickwinkel unabhängige Helligkeitsmessung der Testfelder zu ermöglichen. In dem in Fig. 2 gezeigten Ausführungsbeispiel sind im Dunkel-Referenzfeld 35, in Richtung des Lichtwegs gesehen, nacheinander ein Blockfilter, ein Anregungsfilter für durch 5-ALA induzierte Fluoreszenz (ALA-Anregungsfilter), ein Autofluoreszenz (AF) - Beobachtungsfilter und eine Streuscheibe 38 angeordnet. Das Testfeld 36 enthält eine Anordnung aus Blockfilter, ALA-Anregungfilter und Streuscheibe, das Testfeld 36' aus Blockfilter, AF-Anregungsfilter und Streuscheibe. Die Hell-Referenzfelder 34, 34' und 34" enthalten jeweils nur ein Blockfilter und eine Streuscheibe. Hierdurch sind bestimmte Filtercharakteristiken an der durch das Endoskop beobachtbaren Flächenhelligkeit der Test- und Referenzfelder erkennbar.

Zur Durchführung der Prüfung eines Endoskops wird gemäß Fig. 3 das Endoskop 50 mit dem distalen Ende des Schafts 51 in die Prüfvorrichtung 1 eingesetzt. An den Lichtanschluss 21 der Prüfvorrichtung 1 wird ein Lichtleitkabel 55 angeschlossen, das das von einer externen Lichtquelle 56 erzeugt Beleuchtungslicht in die Prüfvorrichtung überträgt. Durch den visuellen Einblick 52 des Endoskops 50 kann das Target visuell beobachtet und die Helligkeiten der Test- und Referenzfelder miteinander verglichen werden. An die Okularmuschel 53 kann auch eine Videokamera angeschlossen werden, so dass das Erscheinungsbild des Targets auf einem Bildschirm darstellbar und mit automatischer Bildverarbeitung auswertbar ist. Das gleiche gilt für Endoskope mit integriertem elektronischem Bildaufnehmer. Auch wenn in Fig. 3 ein starres Endoskop dargestellt ist, kann auch ein das distale Ende des Schafts eines flexiblen oder semiflexiblen Endoskops in die Prüfvorrichtung eingesetzt werden.

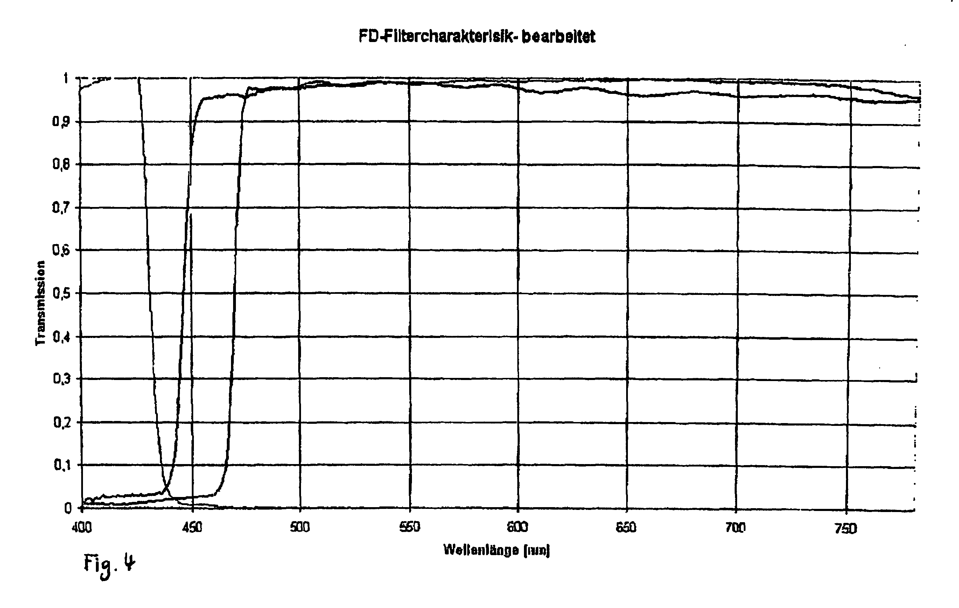

Fig. 4 zeigt die Filtercharakteristiken der bei Autofluoreszenz und ALA-induzierter Fluoreszenz verwendeten Filter. Die Anregung der Fluoreszenz erfolgt jeweils durch kurzwelliges Licht im blauen Bereich des optischen Spektrums, wobei für Autofluoreszenz etwas längerwelliges Licht verwendet wird als für ALA-induzierte Fluoreszenz. Dementsprechend werden im Beleuchtungsstrahlengang Anregungsfilter verwendet, die als Kurzpass-Kantenfilter ausgebildet sind und von einer Transmission von nahezu 100% im kurzwelligen Bereich innerhalb von ca.10 bis 30 nm auf eine Transmission von nahezu 0% abfallen. Dabei ist die Kantenlage des AF-Anregungsfilters mit ca. 450 nm etwas längerwellig als die des ALA-Anregungsfilters mit ca. 430 nm, wie die in Fig. 4 gezeigte Transmissionskurve 60 des AF-Anregungsfilters und die Transmissionskurve 61 des ALA-Anregungsfilters zeigen.

Bei vielen Kantenfiltern ist die Transmission jedoch nicht im gesamten Bereich, der blockiert werden soll, 0%. Vielmehr ergeben sich in einem Abstand von ca. 100 nm von der Kante Transmissionswerte von 10 90%, wie in Fig. 4 dargestellt. Da die verwendeten Lichtquellen, beispielsweise Xenon-Lichtquellen, in der Regel auch in diesem Bereich Licht erzeugen, wird dieses durch einen Blockfilter ausgefiltert, der bei Wellenlängen, die größer als ca. 550 nm sind, die Strahlung abblockt. Für einen solchen Blockfilter gelten allerdings hinsichtlich der Kantenlage weniger strenge Anforderungen als für die Anregungsfilter. Bei einer schmalbandigen Lichtquelle, beispielsweise bei einer Taserlichtquelle, ist ein Blockfilter nicht erforderlich.

Für die möglichst kontrastreiche Beobachtung der vom Gewebe erzeugten Fluoreszenzstrahlung werden Beobachtungsfilter verwendet, die die Anregungsstrahlung nahezu vollständig blockieren, um eine Überstrahlung der schwachen Fluoreszenzstrahlung durch das sehr helle Anregungslicht zur vermeiden. Hierfür sind die Beobachtungsfilter ebenfalls als Kantenfilter ausgebildet, jedoch als Langpassfilter, die bei Wellenlängen, die größer sind als vom Anregungsfilter durchgelassen, durchlässig sind.

Zur Beobachtung des Gewebes im reflektierten Licht kann es vorteilhaft sein, wenn die Transmissionsbereiche der Anregungs- und der Beobachtungsfilter sich in einem geringen Maße überlappen, so dass ein solcher Anteil des vom Gewebe reflektierten oder gestreuten Beleuchtungslichts vom Beobachtungsfilter durchgelassen wird, der zusätzlich zur Fluoreszenzstrahlung beobachtet werden kann, jedoch eine dieser vergleichbare Helligkeit liefert und beispielsweise auch die unspezifische Fluoreszenz des Gewebes kompensieren kann. Im gezeigten Beispiel kann diese reflektierte Strahlung durch ihre blaue Farbe von der im grünen oder roten Bereich des optischen Spektrums emittierten AF- bzw. ALA-induzierter Fluoreszenzstrahlung unterschieden werden.

Die Bcobachtungsfilter haben jeweils eine gegenüber den Anregungsfiltern umgekehrte Filtercharakteristik. Dabei ist die Filtercharakteristik des jeweiligen Beobachtungsfilters der des Anregungsfilters angepasst, um eine definierte Überlappung zu gewährleisten. Deshalb unterscheiden sich bei den verschiedenen Fluoreszenzmoden nicht nur die Anregungsfilter, sondern auch die Beobachtungsfilter. So ist gemäß Fig. 4 die Transmissionsfunktion 62 des AF-Beobachtungsfilters gegenüber der Transmissionsfunktion 63 des ALA-Beobachtungsfilters ebenfalls um ca. 30 nm verschoben. Ferner können die Beobachtungsfilter der jeweiligen Wellenlänge der beobachteten Fluoreszenz angepasst sein oder auch deshalb unterschiedlich sein, um je nach Beobachtungsmodus weitere Beleuchtungslichtanteile in anderen Spektralbereichen durchzulassen oder zu blockieren.

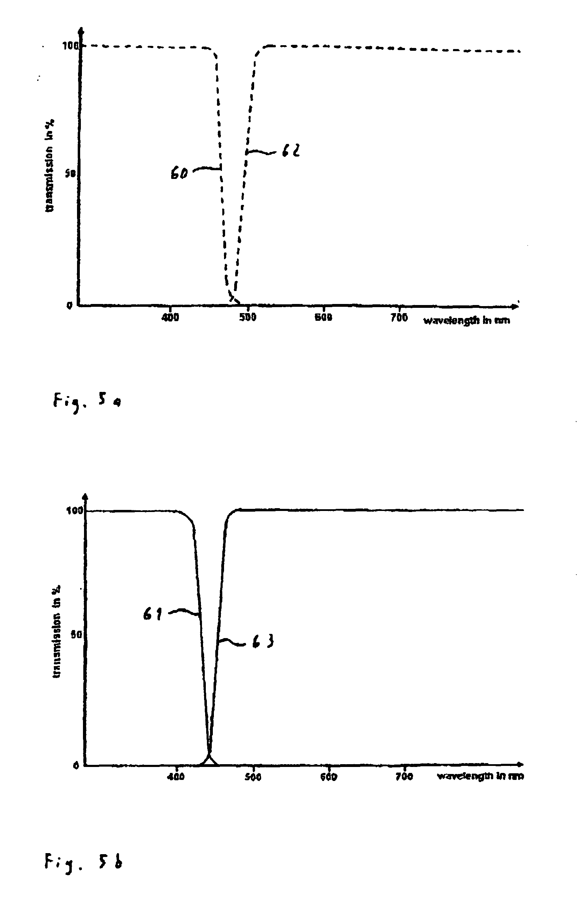

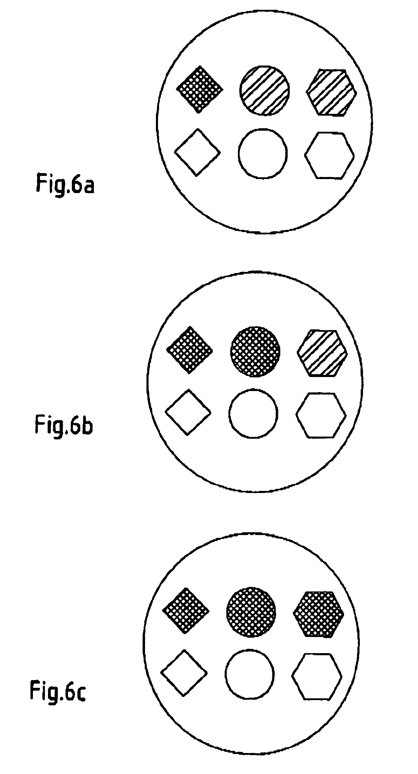

Mit der erfindungsgemäßen Prüfvorrichtung und dem erfindungsgemäßen Prüfverfahren lassen sich die für Autofluoreszenz und für ALA-induzierte Fluoreszenz verwendeten Beobachtungsfilter, die sich in der Kantenlage nur um ca. 30 nm unterscheiden, sicher prüfen und voneinander unterscheiden, ohne die Filter dem Endoskop entnehmen zu müssen. Dies wird anhand von Fig. 5a bis 5d und Fig. 6a bis 6c verdeutlicht.

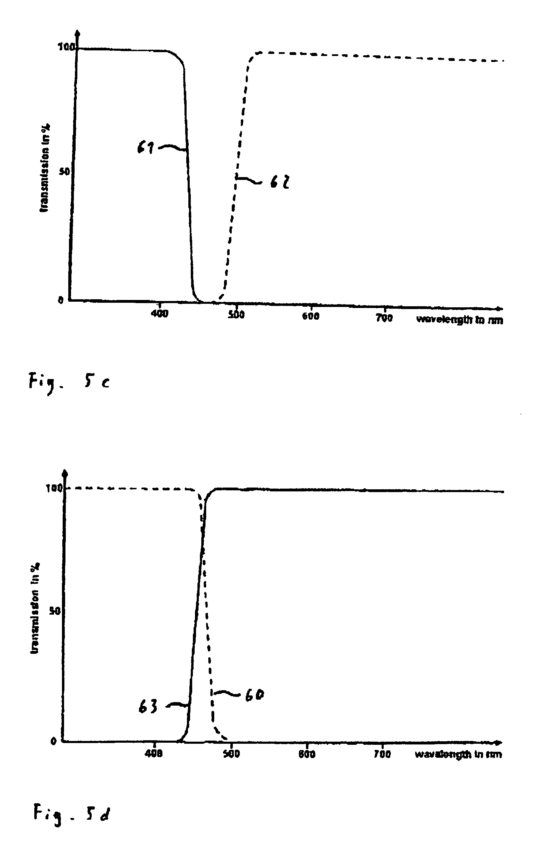

Fig. 5a zeigt die Transmissionskurven 60, 62 des AF-Anregungsfilter und des AF-Beobachtungsfilters. Beide überlappen sich in einem schmalen Bereich mit einer jeweiligen Transmission von wenigen %. Ähnliches gilt für die Transmissionskurven 61, 63 des ALA-Anregungsfilters und des ALA-Beobachtungsfilters, wie in Fig. 5b dargestellt. Andererseits überlappen sich gemäß Fig. 5c die Transmissionskurven 61, 62 des ALA-Anregungsfilters und des AF-Beobachtungsfilters praktisch nicht, so dass bei dieser Filterkombination praktisch kein Licht durchgelassen wird. Demgegenüber überlappen sich die Transmissionskurven 60, 63 des AF-Anregungsfilters und des ALA-Beobachtungsfilters in einem größeren Bereich mit deutlich höherer Transmission, so dass bei dieser Kombination ein erkennbar größerer Lichtanteil transmittiert wird (s. Fig. 5d).

Dies wird erfindungsgemäß zur Prüfung bzw. Unterscheidung der betreffenden Beobachtungsfilter ausgenutzt. Fig. 6a bis 6c zeigt in idealisierter Darstellung das Erscheinungsbild des Targets bei unterschiedlichen im Endoskop vorhandenen Beobachtungsfiltern. Aus Gründen der Übersichtlichkeit sind dabei die Bezugszeichen weggelassen. Die untere Reihe zeigt die Hell-Referenzfelder 34, 34' und 34", die obere Reihe zeigt links die rechteckige Dunkel-Referenz 35, in der Mitte das kreisförmige Testfeld 36 und rechts das sechseckige Testfeld 36'. Wie zu Fig. 2 beschrieben, enthalten die Hell-Referenzfelder lediglich jeweils ein Blockfilter und eine Streuscheibe. Die Hell-Referenzfelder erscheinen daher hell, beispielsweise bei Verwendung einer Xenon-Lichtquelle in hellblauer Farbe, unabhängig davon, ob ein und welches Beobachtungsfilter im geprüflen Endoskop vorhanden ist. Das rechteckige Dunkel-Referenzfeld 35 enthält zusätzlich hierzu die in Fig. 5c gezeigte Kombination aus ALA-Anregungsfilter und AF-Beobachtungsfilter und erscheint daher in jedem Fall nahezu dunkel. Die Referenzfelder können dazu verwendet werden, beispielsweise die Helligkeit der Lichtquelle, die Belichtungszeit und die Empfindlichkeit bzw. die Verstärkung eines elektronischen Bildaufnehmers in einer für die Prüfung geeigneten Weise einzustellen. Die Einstellungen können beispielsweise derart erfolgen, dass die Hcll-Referenzfelder hell erscheinen und dass das rechteckige Dunkel-Referenzfeld gerade noch gegen die schwarze bzw. lichtundurchlässige Fläche des Targets zu erkennen ist.

Ist im zu prüfenden Endoskop kein Beobachtullgsfilter eingebaut, so erscheinen sowohl das kreisförmige Testfeld 36, das zusätzlich zur Streuscheibe und zum Blockfilter ein ALA-Anrcgungsfilter enthält, als auch das sechseckige Testfeld 36', das zusätzlich zur Streuscheibe und zum Blockfilter ein AF-Anregungsfilter enthält, hell. Insbesondere erscheinen beide Testfelder nahezu gleich hell, im beschriebenen Beispiel aufgrund des blauen Anregungslichts bläulich, und deutlich heller als das Dunkel-Referenzfeld 35. Die Hell-Referenzfelder erscheinen hellblau und deutlich heller als die Testfelder 36, 36' (Fig. 6a).

Ist im zu prüfenden Endoskop ein ALA-Beobachtungsfilter vorhanden, so lässt die Kombination mit dem im Testfeld 36 eingebauten ALA-Anregungsfilter gemäß Fig. 5b nur einen geringen Anteil des Lichts hindurch. Andererseits ist die Gesamt-Transmission der Kombination mit dem im Testfeld 36' vorhandenen AF-Anrcgungsfilters gemäß der Überlappung der in Fig. 5d gezeigten Transmissionsfunktionen deutlich höher. In diesem Fall erscheint daher das kreisförmige Testfeld 36 nahezu dunkel, ähnlich dem viereckigen Dunkel-Referenzfeld 35, während das sechseckige Testfcld 36' deutlich heller erscheint, nämlich bläulich, jedoch weniger hell als die hellblaue Hell-Referenzfelder (Fig. 6b).

Ist im zu prüfenden Endoskop ein AF-Beobachtungsfilter vorhanden, so lässt die Kombination mit dem im Testfeld 36 vorhandenen ALA-Anregungsfilter gemäß Fig. 5c praktisch kein Licht hindurch, ebenso wie die Kombination mit dem im Testfeld 36' eingesetzten AF-Anregungsfilter gemäß Fig. 5a nur einen geringen Lichtanteil durchlässe. In diesem Fall erscheinen daher sowohl das runde Testfeld 36 als auch das sechseckige Testfeld 36' nahezu dunkel, ähnlich der Dunkelreferenz 35 (Fig. 6c).

Gemäß dem erfindungsgemäßen Verfahren zur Prüfung eines Fluoreszenzendoskops wird, nachdem die zu Fig. 3 beschriebenen Schritte und bei einem elektronischen Bildaufnehmer ggf. ein Weißabgleich durchgeführt worden sind, das Targets in einer der drei in Fig. 6a bis 6c dargestellten Erscheinungsformen beobachlet. Je nach Erscheinungsbild der Referenzfelder sind die Einstellung der Lichtquelle sowie Belichtungszeit bzw. Integrationszeit einer Kamera zu korrigieren. Durch einen Vergleich der Helligkeiten der Testfelder 36 und 36' untereinander sowie mit dem Dunkel-Referenzfeld 35 und den Hell-Referenzfeldern 34, 34', 34" ergibt sich eindeutig, ob und ggf. welches Filter im Endoskop vorhanden ist. Auf diese Weise ist durch eine einfache Blickkontrolle eine sichere Unterscheidung der verschiedenen Varianten eines Fluoreszenzendoskops bzw. der verschiedenen Beobachtungsmoden eines umschaltbaren Fluoreszenzendoskops möglich.

Bezugszeichenliste

| | |
|---|---|
| 1 | Prüfvorrichtung |
| 2 | Gehäuse |
| 3 | Schraube |
| 4 | Fuß |
| 5 | Längsachse |
| 6 | Hohlraum |
| 7 | Hülse |
| 10 | Adapter |
| 11 | Bohrung |
| 12 | Fase |
| 13 | Platte |
| 14 | Bohrung |
| 15 | Bohrung |
| 20 | Lichtanschlussplatte |
| 21 | Lichtanschluss |
| 30 | Filtermodul |
| 31 | Erste Deckplatte |
| 32 | Zweite Deckplatte |
| 33 | Scheibe |
| 34, 34', 34" | Hell-Referenzfeld |
| 35 | Dunkel-Referenzfeld |
| 36, 36' | Testfeld |
| 37 | Filter |
| 38 | Streuscheibe |
| 39 | Durchbruch |
| 40 | Linsenmodul |
| 50 | Endoskop |

51          Schaft

52          Visueller Einblick

53          Okularmuschel

55          Lichtleitkabel

56          Lichtquelle

60          Transmissionskurve des AF-Anregungsfilters

61          Transmissionskurve des ALA-Anregungsfilters

62          Transmissionskurve des AF-Beobachtungsfilters

63          Transmissionskurve des ALA-Beobachtungsfilters

## Patentansprüche

1.  Kombination eines Endoskops, das eine Beobachtungsfilteranordnung mit einer vorgegebenen spektralen Soll-Charakteristik aufweist, und einer Vorrichtung zur Prüfung des Endoskops, wobei die Vorrichtung ein Target mit mindestens einem Testfeld (36, 36') sowie eine Halterung zur Aufnahme des Endoskops zur Beobachtung des mindestens einen Testfelds aus einer ersten Richtung umfasst und wobei das Testfeld eine Testfilteranordnung enthält, die aus einer zweiten Richtung beleuchtbar ist, so dass dem Testfeld eine mit dem Endoskop beobachtbare Helligkeit im durch die Testfilteranordnung transmittierten Licht zugeordnet werden kann, **dadurch gekennzeichnet, dass** die spektrale Charakteristik der Testfilteranordnung zu der Soll-Charakteristik der Beobachtungsfilteranordnung umgekehrt ist.

2.  Kombination eines Endoskops für Fluoreszenzbeobachtungen, das eine Beobachtungsfilteranordnung mit einer vorgegebenen spektralen Soll-Charakteristik aufweist, und einer Vorrichtung zur Prüfung des Endoskops, wobei dem Endoskop eine Beleuchtungsfilteranordnung zugeordnet ist, wobei die Vorrichtung ein Target mit mindestens einem Testfeld (36, 36') sowie eine Halterung zur Aufnahme des Endoskops zur Beobachtung des mindestens einen Testfelds aus einer ersten Richtung umfasst und wobei das Testfeld eine Testfilteranordnung enthält, die aus einer zweiten Richtung beleuchtbar ist, so dass dem Testfeld eine mit dem Endoskop beobachtbare Helligkeit im durch die Testfilteranordnung transmittierten Licht zugeordnet werden kann, **dadurch gekennzeichnet, dass** die spektrale Charakteristik der Testfilteranordnung der spektralen Charakteristik der Beleuchtungsfilteranordnung entspricht.

3.  Kombination nach Anspruch 1 oder 2, **dadurch gekennzeichnet, dass** die Testfilteranordnung des mindestens einen Testfelds aus einer Mehrzahl von Filtern (37) besteht.

4.  Kombination nach einem der vorhergehenden Ansprüche, **dadurch gekennzeichnet, dass** das Target mindestens ein Referenzfeld (34, 34', 34", 35) aufweist.

5.  Kombination nach einem der vorhergehenden Ansprüche, **dadurch gekennzeichnet, dass** das Target eine Mehrzahl von Testfeldern (36, 36') aufweist, die jeweils eine Testfilteranordnung enthalten.

6.  Kombination nach Anspruch 5, **dadurch gekennzeichnet, dass** das die spektralen Charakteristiken der Testfilteranordnungen mindestens zweier Testfelder (36, 36') voneinander unterschiedlich sind.

7.  Kombination nach einem der Ansprüche 4 bis 6, **dadurch gekennzeichnet, dass** die Test- und/oder Referenzfelder unterschiedliche geometrische Formen aufweisen.

8.  Kombination nach einem der Ansprüche 4 bis 7, **dadurch gekennzeichnet, dass** eine Mehrzahl von Test- und/oder Referenzfeldern von einer gemeinsamen Lichtquelle beleuchtbar ist.

9.  Kombination nach einem der vorhergehenden Ansprüche, **dadurch gekennzeichnet, dass** das Target lichtundurchlässig und mindestens ein Test- und/oder Referenzfeld als lichtdurchlässiger Bereich des Targets ausgebildet ist.

10. Kombination nach Anspruch 9, **dadurch gekennzeichnet, dass** die Vorrichtung ein Linsenmodul (40) umfasst.

11. Kombination nach einem der vorhergehenden Ansprüche, **dadurch gekennzeichnet, dass** in mindestens einem Test- und/oder Referenzfeld eine Streuscheibe (38) vorhanden ist.

12. Kombination nach einem der vorhergehenden Ansprüche, **dadurch gekennzeichnet, dass** die Vorrichtung ein lichtdichtes Gehäuse (2) umfasst, das einen Hohlraum (6) umschließt, in dem das Target angeordnet ist und der durch die Halterung lichtdicht abgeschlossen ist.

13. Kombination nach Anspruch 12, **dadurch gekennzeichnet, dass** die Halterung als auswechselbarer Adapter (10) ausgebildet ist, der in das lichtdichte Gehäuse (2) einsetzbar ist.

**14.** Verfahren zur Prüfung eines Endoskops, das eine Beobachtungsfilteranordnung mit einer vorgegebenen spektralen Soll-Charakteristik aufweist, umfassend folgende Schritte:

- Bereitstellen eines Targets mit mindestens einem Testfeld (36, 36'), das eine Testfilteranordnung enthält, wobei die spektrale Charakteristik der Testfilteranordnung zu der Soll-Charakteristik der Beobachtungsfilteranordnung umgekehrt ist,
- Anordnen des Endoskops in einer Halterung zur Beobachtung des mindestens einen Testfelds aus einer ersten Richtung,
- Beleuchten der Testfilteranordnung aus einer zweiten Richtung,
- Bestimmen einer Helligkeit des Testfelds mit dem Endoskop, und
- Vergleichen der Helligkeit des Testfelds mit einer Soll-Helligkeit.

**15.** Verfahren zur Prüfung eines Endoskops für Fluoreszenzbeobachtungen, das eine Beobachtungsfilteranordnung mit einer vorgegebenen spektralen Soll-Charakteristik aufweist, wobei dem Endoskop eine Beleuchtungsfilteranordnung zugeordnet ist, umfassend folgende Schritte:

- Bereitstellen eines Targets mit mindestens einem Testfeld (36, 36'), das eine Testfilteranordnung enthält, wobei die spektrale Charakteristik der Testfilteranordnung der spektralen Charakteristik der Beleuchtungsfilteranordnung entspricht,
- Anordnen des Endoskops in einer Halterung zur Beobachtung des mindestens einen Testfelds aus einer ersten Richtung,
- Beleuchten der Testfilteranordnung aus einer zweiten Richtung,
- Bestimmen einer Helligkeit des Testfelds mit dem Endoskop, und
- Vergleichen der Helligkeit des Testfelds mit einer Soll-Helligkeit.

**Claims**

**1.** Combination of an endoscope, having an observation filter arrangement with a predetermined spectral intended characteristic, and an apparatus for checking the endoscope, the apparatus comprising a target with at least one test field (36, 36') and a holder for holding the endoscope in order to observe the at least one test field from a first direction, and the test field containing a test filter arrangement that can be illuminated from a second direction such that a brightness in the light, which is transmitted through the test filter arrangement and can be observed by the endoscope, can be associated with the test field, **characterized in that** the spectral characteristic of the test filter arrangement is inverted with respect to the intended characteristic of the observation filter arrangement.

**2.** Combination of an endoscope for fluorescence observations, having an observation filter arrangement with a predetermined spectral intended characteristic, and an apparatus for checking the endoscope, with an illumination filter arrangement being associated with the endoscope, the apparatus comprising a target with at least one test field (36, 36') and a holder for holding the endoscope in order to observe the at least one test field from a first direction, and the test field containing a test filter arrangement that can be illuminated from a second direction such that a brightness in the light, which is transmitted through the test filter arrangement and can be observed by the endoscope, can be associated with the test field, **characterized in that** the spectral characteristic of the test filter arrangement corresponds to the spectral characteristic of the illumination filter arrangement.

**3.** Combination according to Claim 1 or 2, **characterized in that** the test filter arrangement of the at least one test field consists of a plurality of filters (37).

**4.** Combination according to one of the preceding claims, **characterized in that** the target has at least one reference field (34, 34', 34", 35).

**5.** Combination according to one of the preceding claims, **characterized in that** the target has a plurality of test fields (36, 36'), which each contain one test filter arrangement.

**6.** Combination according to Claim 5, **characterized in that** the spectral characteristics of the test filter arrangements of at least two test fields (36, 36') differ from one another.

**7.** Combination according to one of Claims 4 to 6, **characterized in that** the test and/or reference fields have different geometric shapes.

**8.** Combination according to one of Claims 4 to 7, **characterized in that** a plurality of test and/or reference fields can be illuminated by a common light source.

**9.** Combination according to one of the preceding claims, **characterized in that** the target is opaque and at least one test and/or reference field is embodied as a light-permeable region of the target.

**10.** Combination according to Claim 9, **characterized in that** the apparatus comprises a lens module (40).

**11.** Combination according to one of the preceding claims, **characterized in that** a diffusion screen (38) is present in at least one test and/or reference field.

**12.** Combination according to one of the preceding claims, **characterized in that** the apparatus comprises a lightproof housing (2), which encloses a cavity (6) in which the target is arranged and which is completed in a lightproof manner by the holder.

**13.** Combination according to Claim 12, **characterized in that** the holder is embodied as replaceable adapter (10) which can be inserted into the lightproof housing (2).

**14.** Method for checking an endoscope with an observation filter arrangement with a predetermined spectral intended characteristic, comprising the following steps:

- providing a target with at least one test field (36, 36') containing a test filter arrangement, the spectral characteristic of the test filter arrangement being inverted with respect to the intended characteristic of the observation filter arrangement,
- arranging the endoscope in a holder in order to observe the at least one test field from a first direction,
- illuminating the test filter arrangement from a second direction,
- determining a brightness of the test field using the endoscope, and
- comparing the brightness of the test field with an intended brightness.

**15.** Method for checking an endoscope for fluorescence observations with an observation filter arrangement with a predetermined spectral intended characteristic, with an illumination filter arrangement being associated with the endoscope, comprising the following steps:

- providing a target with at least one test field (36, 36') containing a test filter arrangement, the spectral characteristic of the test filter arrangement corresponding to the spectral characteristic of the illumination filter arrangement,
- arranging the endoscope in a holder in order to observe the at least one test field from a first direction,
- illuminating the test filter arrangement from a second direction,
- determining a brightness of the test field using the endoscope, and

- comparing the brightness of the test field with an intended brightness.

**Revendications**

**1.** Combinaison formée
d'un endoscope doté d'un ensemble de filtres d'observation dont la caractéristique spectrale de consigne est prédéterminée et
d'un dispositif de vérification de l'endoscope,
le dispositif comportant une cible dotée d'au moins un champ de test (36, 36') ainsi qu'un support qui reprend l'endoscope pour observer le ou les champs de test dans une première direction,
le ou les champs de test contenant un ensemble de filtres de test qui peut être éclairé dans une deuxième direction de telle sorte que la luminosité de la lumière transmise à travers l'ensemble de filtres de test et observée à l'aide de l'endoscope puisse être associée au champ de test,
**caractérisée en ce que**
la caractéristique spectrale de l'ensemble de filtres de test est l'inverse de la caractéristique de consigne de l'ensemble de filtres d'observation.

**2.** Combinaison formée
d'un endoscope prévu pour l'observation en fluorescence et doté d'un ensemble de filtres d'observation dont la caractéristique spectrale de consigne est prédéterminée et
d'un dispositif de vérification de l'endoscope,
un ensemble de filtres d'éclairage étant associé à l'endoscope,
le dispositif comportant une cible dotée d'au moins un champ de test (36, 36') ainsi qu'un support qui reprend l'endoscope pour observer le ou les champs de test dans une première direction,
le ou les champs de test contenant un ensemble de filtres de test qui peut être éclairé dans une deuxième direction de telle sorte que la luminosité de la lumière transmise à travers l'ensemble de filtres de test et observée à l'aide de l'endoscope puisse être associée au champ de test,
**caractérisée en ce que**
la caractéristique spectrale de l'ensemble de filtres de test correspond à la caractéristique spectrale de l'ensemble de filtres d'éclairage.

**3.** Combinaison selon les revendications 1 ou 2, **caractérisée en ce que** l'ensemble de filtres de test du ou des champs de test est constitué de plusieurs filtres (37).

**4.** Combinaison selon l'une des revendications précédentes, **caractérisée en ce que** la cible présente au moins un champ de référence (34, 34', 34", 35).

**5.** Combinaison selon l'une des revendications précédentes, **caractérisée en ce que** la cible présente plusieurs champs de test (36, 36') qui contiennent chacun un ensemble de filtres de test.

**6.** Combinaison selon la revendication 5, **caractérisée en ce que** les caractéristiques spectrales des ensembles de filtre de test d'au moins deux champs de test (36, 36') diffèrent les unes des autres.

**7.** Combinaison selon l'une des revendications 4 à 6, **caractérisée en ce que** les champs de test et/ou les champs de référence ont des formes géométriques différentes.

**8.** Combinaison selon l'une des revendications 4 à 7, **caractérisée en ce que** plusieurs champs de test et/ou champs de référence peuvent être éclairés par une source de lumière commune.

**9.** Combinaison selon l'une des revendications précédentes, **caractérisée en ce que** la cible est transparente et **en ce qu'**au moins un champ de test et/ou un champ de référence sont configurés comme parties transparentes de la cible.

**10.** Combinaison selon la revendication 9, **caractérisée en ce que** le dispositif comporte un module de lentilles (40).

**11.** Combinaison selon l'une des revendications précédentes, **caractérisée en ce qu'**un disque de diffusion (38) est prévu dans au moins un champ de test et/ou champ de référence.

**12.** Combinaison selon l'une des revendications précédentes, **caractérisée en ce que** le dispositif comporte un boîtier opaque (2) qui entoure une cavité (6) dans laquelle est disposée la cible et que le support ferme de manière opaque à la lumière.

**13.** Combinaison selon la revendication 12, **caractérisée en ce que** le support est configuré comme adaptateur (10) remplaçable qui peut être inséré dans le boîtier opaque (2).

**14.** Procédé de test d'un endoscope qui présente un ensemble de filtres d'observation dont la caractéristique spectrale de consigne est prédéterminée, le procédé comportant les étapes suivantes :

   - prévoir une cible qui présente au moins un champ de test (36, 36') qui contient un ensemble de filtres de test, la caractéristique spectrale de l'ensemble de filtres de test étant l'inverse de la caractéristique de consigne de l'ensemble de filtres d'observation,
   - placer l'endoscope dans un support pour observer le ou les champs de test dans une première direction,
   - éclairer l'ensemble de filtres de test dans une deuxième direction,
   - déterminer la luminosité du champ de test à l'aide d'un endoscope et
   - comparer la luminosité du champ de test à une valeur de consigne de luminosité.

**15.** Procédé de test d'un endoscope prévu pour des observations en fluorescence et qui présente un ensemble de filtres d'observation dont la caractéristique spectrale de consigne est prédéterminée, un ensemble de filtres d'éclairage étant associé à l'endoscope, le procédé comportant les étapes suivantes :

   - prévoir une cible qui présente au moins un champ de test (36, 36') qui contient un ensemble de filtres de test, la caractéristique spectrale de l'ensemble de filtres de test correspondant à la caractéristique spectrale de l'ensemble de filtres d'éclairage,
   - placer l'endoscope dans un support pour observer le ou les champs de test dans une première direction,
   - éclairer l'ensemble de filtres de test dans une deuxième direction,
   - déterminer la luminosité du champ de test à l'aide d'un endoscope et
   - comparer la luminosité du champ de test à une valeur de consigne de luminosité.

Fig.1a

Fig.1b

Fig. 2

Fig. 3

FD-Filtercharakterisik- bearbeitet

Fig. 4

Fig. 5 a

Fig. 5 b

Fig. 5c

Fig. 5d

Fig.6a

Fig.6b

Fig.6c

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9711636 A
- US 5142359 A
- WO 9811945 A